Europäisches Patentamt

European Patent Office

Office européen des brevets

(11) Numéro de publication: **0 433 834 A1**

(12) **DEMANDE DE BREVET EUROPEEN**

(21) Numéro de dépôt: 90123719.8

(22) Date de dépôt: 10.12.90

(51) Int. Cl.5: **A61F 9/00, A61B 3/125**

(30) Priorité: 21.12.89 CH 4595/89
22.01.90 FR 9000778

(43) Date de publication de la demande:
26.06.91 Bulletin 91/26

(84) Etats contractants désignés:
AT BE DE GB IT LU NL SE

(71) Demandeur: LASAG AG
Mittlere Strasse 52
CH-3600 Thun(CH)

(72) Inventeur: Dürr, Ulrich
Ziegeleistrasse 38
CH-3612 Steffisburg(CH)
Inventeur: Henchoz, Pierre David
Chemin A.-Burnat 20
CH-1814 La Tour de Peilz(CH)

(74) Mandataire: Caron, Gérard et al
ICB Ingénieurs Conseils en Brevets SA
Passage Max. Meuron 6
CH-2001 Neuchâtel(CH)

(54) Verre de contact pour l'ophtalmothérapie transsclérotique / transchoroidal par faisceau lumineux, et notamment par rayon laser.

(57) L'invention concerne un verre de contact pour l'ophtalmothérapie.

Ce verre de contact, qui comprend au moins une lentille optique (12) pourvue d'une surface d'entrée (22) d'un faisceau lumineux et d'une surface de sortie (24) dont au moins une partie est destinée à venir en contact directement avec la sclérotique (1) de l'oeil et par laquelle sort le faisceau lumineux, est caractérisé en ce que la surface de sortie (24) comporte un élément protubérant (18) qui est apte à transmettre le faisceau lumineux et qui est destiné à venir comprimer la sclérotique pour diminuer son épaisseur.

Ce verre de contact s'applique à l'ophtalmothérapie transsclérotique / transchoroïdale.

Fig.2

## VERRE DE CONTACT POUR L'OPHTALMOTHERAPIE TRANSSCLEROTIQUE / TRANSCHOROIDALE PAR FAISCEAU LUMINEUX, ET NOTAMMENT PAR RAYON LASER

L'invention concerne un verre de contact pour l'ophtalmothérapie réalisée à l'aide de faisceaux lumineux.

Plus particulièrement, cette invention concerne un verre de contact destiné au traitement transsclérotique et éventuellement transchoroïdal de l'oeil, c'est-à-dire à l'irradiation de certains tissus de l'oeil au travers de la sclérotique, et éventuellement de la choroïde, et ce notamment par un procédé de photocoagulation.

Ce type de traitement est réalisé par l'irradiation de la ou des régions visées de l'oeil par un faisceau intense de lumière notamment de lumière cohérente, telle qu'un rayon laser.

On connaît déjà, d'après notamment le document CH-A-640 401, un verre de contact destiné à l'ophtalmothérapie par rayon laser. Toutefois, ce type de verre de contact, bien que donnant satisfaction dans l'application pour laquelle il est destiné, c'est-à-dire pour des traitements transpupillaires, ne peut malheureusement pas être utilisé pour un traitement en dehors de l'axe visuel de l'oeil, comme cela est nécessaire dans le cas des thérapies transsclérotiques / transchoroïdales.

Par ailleurs, on connaît d'après la publication du "American Journal of Ophtalmology", volume 108, numéro 4, page 457 d'octobre 1989, un verre de contact particulièrement destiné aux interventions transsclérotiques à l'aide d'un laser du type Nd : YAG.

Ce verre de contact est constitué ,d'une part, d'une lentille optique dont une région est destinée à transmettre le faisceau lumineux, et, d'autre part, d'une partie support qui s'étend autour de la lentille et qui comporte une région découpée apte à recevoir l'arcade sourcilière.

La forme de cette lentille est telle qu'elle épouse sensiblement la surface extérieure de l'oeil. Sa partie centrale sphérique concave qui vient s'appliquer en regard de la cornée est opaque pour éviter toute entrée de lumière parasite. Elle est prolongée par une partie périphérique tronconique destinée à venir s'appliquer sur la sclérotique. C'est au travers de cette partie périphérique, qui comporte une série de repères, que doivent passer les faisceaux lumineux pour atteindre, à travers la sclérotique et éventuellement la choroïde, les tissus visés de l'oeil.

Toutefois, cette lentille est d'une forme compliquée et elle est par conséquent coûteuse. En outre, on sait qu'un faisceau lumineux est fortement dispersé en traversant la sclérotique, et que cette dipersion est d'autant plus importante que cette sclérotique est épaisse.

Le verre de contact susmentionné ne permet pas une modification substantielle de cette caractéristique optique de la sclérotique. Aussi, lorsqu'il est utilisé, le faisceau lumineux ne peut pas être transmis efficacement sur le point à traiter.

Aussi, l'invention a-t-elle pour but de remédier à ces inconvénients en fournissant un verre de contact qui permette le traitement de l'oeil par une stratégie transsclérotique / transchoroïdale à l'aide d'un faisceau lumineux et qui soit susceptible d'influer de façon significative sur les propriétés optiques de la sclérotique.

Elle a aussi pour but de fournir un verre de contact simple et peu coûteux, facilement manipulable par les praticiens.

L'invention a donc pour objet un verre de contact pour l'ophtalmothérapie transsclérotique / transchoroïdale a l'aide d'un faisceau lumineux, comprenant au moins une lentille optique comportant une surface d'entrée du faisceau lumineux et une surface de sortie dont au moins une partie est destinée à venir en contact directement avec la conjonctive ou la sclérotique de l'oeil à traiter et par laquelle sort ledit faisceau lumineux, caractérisé en ce que la surface de sortie de ladite lentille comporte un élément protubérant apte à transmettre ledit faisceau lumineux et destiné à venir comprimer la sclérotique afin de diminuer son épaisseur à l'endroit du passage du faisceau lumineux et d'augmenter la tranmission de ce faisceau.

Suivant une autre caractéristique de l'invention, l'élément protubérant a une surface frontale faible par rapport à la surface frontale de l'oeil à traiter.

On précisera aussi que, de préférence, l'élément protubérant vient de matière avec ladite lentille optique.

L'invention sera mieux comprise à la lecture de la desciption détaillée qui suit, prise en référence aux dessins annexés qui sont donnés uniquement à titre d'exemple, et dans lesquels :

- la figure 1 est une vue en coupe d'un oeil humain dont des régions particulières rp doivent être traitées à l'aide du verre de contact selon l'invention;

- la figure 2 est une vue en coupe du verre de contact selon l'invention dans sa position lors du traitement, par un faisceau laser, d'une région rp de l'oeil de la figure 1, ce verre de contact étant pourvu d'un moyen support selon un premier mode de réalisation;

- la figure 3 est une vue de côté d'une lentille optique équipant le verre selon l'invention, selon un premier mode de réalisation;

- les figures 4 à 11 sont des vues de côté d'autres modes de réalisation de la lentille optique selon l'invention;

- la figure 12 est une vue en coupe d'un verre de contact en position de repos équipé d'un moyen support selon un deuxième mode de réalisation;
- La figure 12a est une vue détaillée d'un moyen de réglage de la pression fournie par des organes élastiques équipant le verre de contact de la figure 12;
- la figure 13 est une vue en coupe d'un verre de contact selon l'invention, équipé d'un moyen support selon un troisième mode de réalisation, cet ensemble étant en position active; et
- la figure 14 est une vue similaire à la figure 13, mais représentant l'ensemble lentille-moyen support dans sa position de repos.

Avant d'expliquer de façon détaillée les caractéristiques de l'invention, on rappellera quelles sont les parties essentielles de l'oeil humain pour mieux situer et comprendre le domaine d'application du verre de contact selon l'invention.

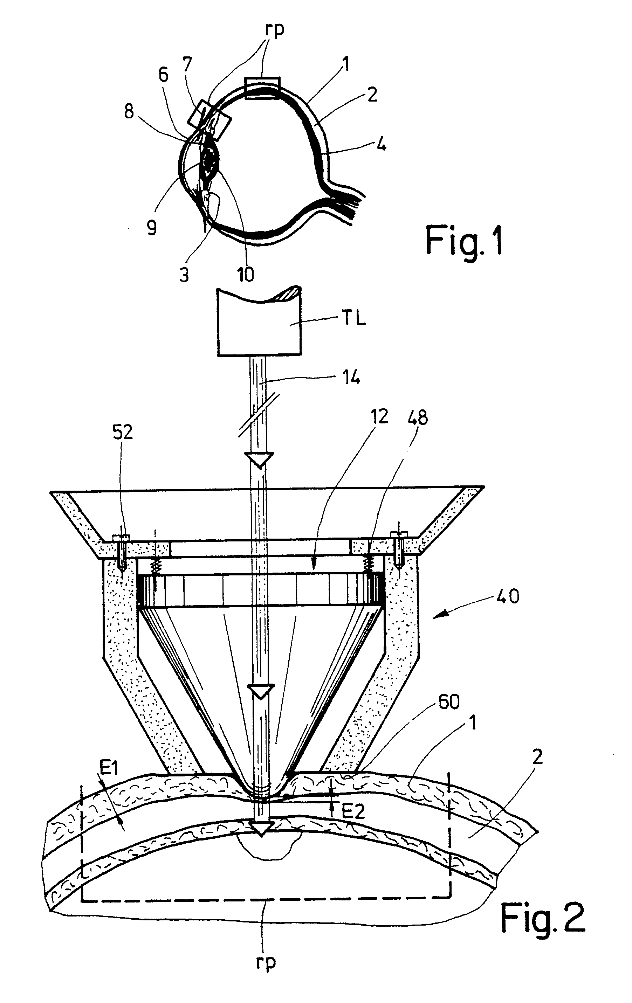

L'oeil humain, tel qu'il est représenté à la figure 1, comporte une enveloppe ou coque extérieure sensiblement sphérique 1 appelée sclérotique, tapissée intérieurement d'une membrane vasculaire 2, la choroïde. Sous cette choroïde 2 se trouve la rétine 4 qui est une membrane nerveuse faisant fonction de plaque sensible. Au niveau de la partie frontale et centrale de l'oeil, la sclérotique 1 est remplacée par la cornée 6 sous laquelle sont logés l'iris 8, la pupille 9 et le cristallin 10. A l'extérieur et sur le pourtour de la cornée 6 se trouve la conjonctive 7 qui recouvre partiellement la sclérotique et qui forme l'étanchéité entre l'oeil et la cavité orbitale (non représenté).

Le verre de contact selon l'invention, qui est représenté dans sa position active et selon un premier mode de réalisation à la figure 2, est destiné à l'irradiation de certains tissus de l'oeil, tels que par exemple la rétine 4 ou le corps ciliaire 3, au travers de la sclérotique 1 mais aussi au travers de la choroïde 2, par des thérapies appelées transsclérotiques / transchoroïdales. Il permet donc le traitement de tissus se trouvant dans des régions rp situées sous la sclérotique et éventuellement sous la conjonctive, par un accès autre que la cornée. Ce verre de contact (figure 2) est constitué, d'une part, d'une lentille optique 12 apte à transmettre un faisceau lumineux 14 émis par la tête TL d'un dispositif laser non représenté, et d'autre part, d'un moyen support 40 dans lequel est logée la lentille 12.

Ce dispositif laser est de préférence du type Nd : YAG, mais peut être constitué par d'autres dispositifs susceptibles de fournir un faisceau de lumière intense.

L'ophtalmothérapie par faisceau laser est une technique aujourd'hui largement connue et sa mise en oeuvre, ainsi que les phénomènes thérapeutiques qu'elle engendre ne seront pas décrits ici de façon plus détaillée.

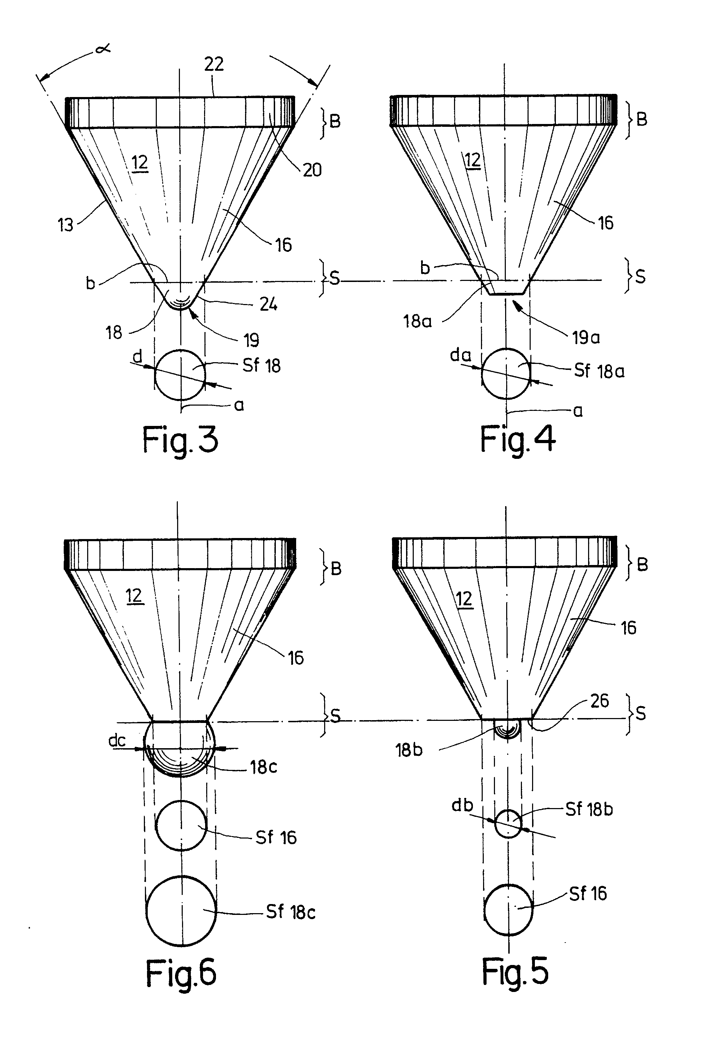

Comme on le voit sur la figure 3, la lentille optique 12 est constituée d'un corps 16 pourvu à son sommet S d'un élément protubérant 18.

De préférence, l'élément protubérant 18 vient de matière avec le corps 16 et il est réalisé en même temps que celui-ci.

Ainsi, la lentille optique 12 et l'élément protubérant 18 forment une pièce monolithique.

Dans les modes de réalisation des figures 3 à 11, le corps 16 de la lentille 12 est de forme sensiblement tronconique.

L'angle a au sommet du cône du corps 16 est choisi de sorte qu'il soit plus grand que l'angle du cône formé par le faisceau de traitement focalisé. Bien que cette forme tronconique du corps 16 soit la seule représentée et constitue la forme préférée, on précisera que le corps 16 peut être de toute autre forme, par exemple cylindrique, sphérique ou polyédrique.

Le corps 16 est surmonté à sa base B par une partie de guidage mécanique 20 venant de matière avec ledit corps et de forme sensiblement cylindrique. La face plane extérieure 22 de la partie de guidage 20 forme, sur la lentille 12, une surface d'entrée du faisceau lumineux 14, tandis que l'extrémité opposée 19 de la lentille 12, au niveau du sommet S, forme une surface de sortie 24 de ce faisceau.

De préférence, la partie de guidage 20, le corps 16, et l'élément protubérant 18 sont disposés de façon coaxiale, leurs surfaces latérales respectives ayant un axe commun référencé a.

Par ailleurs, dans les deux modes de réalisation de l'élément protubérant 18 représentés aux figures 3 et 4, cet élément 18 comporte une surface latérale de forme sensiblement tronconique qui constitue en partie la surface de sortie 24 et qui est ménagée exactement dans le prolongement du corps 16, avec un même angle au sommet que celui-ci.

Ainsi, la base b de l'élément protubérant 18 est accolée voire confondue avec le sommet S du corps 16 de la lentille 12.

Dans le mode de réalisation de la figure 3, l'élément protubérant 18 présente une extrémité libre 19, formant la pointe de la lentille 12, de forme partiellement sphérique, et plus particulièrement de forme sensiblement hémisphérique. Cette extrémité libre sphérique 19 est, dans ce mode de réalisation, parfaitement raccordée à, et dans le prolongement de la surface latérale tronconique de l'élément protubérant 18 et elle forme avec cette dernière la surface de sortie 24.

Selon un deuxième mode de réalisation représenté à la figure 4, l'extrémité libre 19a de l'élément protubérant 18a a une forme sensiblement plane et constitue une surface plane circulaire perpendiculaire à l'axe commun a.

Dans un troisième mode de réalisation représenté à la figure 5, l'élément protubérant 18b est ménagé au centre d'une surface plane 26 normale à l'axe a et constituant l'extrémité du corps 16, au niveau de son sommet S.

Dans cet exemple, l'élément protubérant 18b est de forme hémisphérique et il a un diamètre db tel que sa surface frontale Sf18b est plus petite que la surface frontale Sf16 de l'extrémité libre correspondante du corps 16.

Ainsi, et comme on le comprendra ci-après, la surface annulaire 26 qui entoure l'élément protubérant 18b et qui forme l'extrémité libre du corps 16 au niveau de son sommet S, constitue une butée de positionnement et de profondeur destinée à venir en contact avec la conjonctive 7 ou avec la sclérotique 1 de l'oeil.

Dans un autre mode de réalisation représenté à la figure 6, l'élément protubérant 18c est constitué par les trois quarts d'une sphère dont le diamètre dc est supérieur à celui de l'extrémité libre correspondante du corps 16 formant son sommet S.

Cet élément protubérant 18c forme un renflement en forme de goutte d'eau au niveau du sommet du corps 16 de la lentille 12, c'est-à-dire au niveau de son extrémité de plus faible section.

Quoi qu'il en soit, on précisera ici que dans tous ces modes de réalisation, la surface frontale de l'élément protubérant 18, 18a, 18b, 18c, c'est-à-dire sa projection sur un plan normal à l'axe longitudinal a, est largement inférieure à celle de l'oeil à traiter, ce qui permet de comprimer la sclérotique 1 et de diminuer son épaisseur en appliquant une force limitée.

A titre d'exemple, le diamètre caractéristique d, da, db et dc de l'élément protubérant peut être compris entre 100 " $10^{-6}$ mètre (100 micromètres) et quelques millimètres.

Toutefois, et pour des raisons que l'on expliquera ci-après, ce diamètre, et par conséquent la surface frontale de l'élément protubérant seront choisis de manière à ne pas blesser la sclérotique 1, et ce en fonction de la pression autorisée sur la région rp de l'oeil.

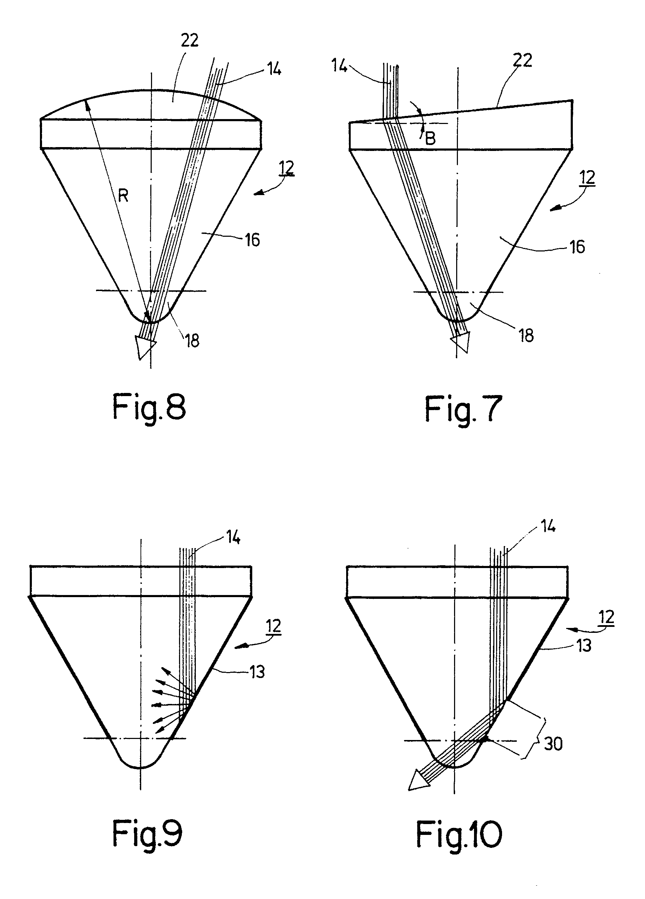

Selon les modes de réalisation des figures 3 à 6, la surface d'entrée 22 du faisceau lumineux dans la lentille 12 est de forme sensiblement plane et elle est disposée orthogonalement à l'axe commun a. Dans un autre mode de réalisation, représenté à la figure 7, cette surface d'entrée 22 est aussi de forme sensiblement plane, mais légèrement inclinée par rapport à l'axe longitudinal a de la lentille 12. Dans encore un autre mode réalisation représenté à la figure 8, la surface d'entrée 22 est constituée par une surface sphérique de rayon de courbure R sensiblement égal à la distance entre cette surface et l'extrémité libre de l'élément protubérant 18 ou sensiblement égal à la distance entre cette surface et le point de focalisation du faisceau appliqué. Ainsi, à chaque point de la surface 22, le faisceau lumineux 14 est orthogonal à la tangente de ce point sur la surface 22, ce qui ne modifie pas la position du point de focalisation en cas de mouvement inopportun de la lentille 12 sur l'oeil. Bien entendu, la forme de ces surfaces d'entrée 22 a une conséquence directe sur l'orientation du faisceau lumineux 14 à l'intérieur de la lentille 12 et elle sera choisie en fonction de la situation dans l'oeil de la région à irradier par rapport à l'orientation du faisceau laser 14.

On précisera aussi que la fonction du corps 16 de la lentille 12 est de transmettre le faisceau lumineux 14 jusqu'à l'élément protubérant 18. La surface d'entrée 22 est de préférence revêtue d'un revêtement anti-réfléchissant pour les longueurs d'onde des faisceaux de visée et de traitement fournis par la tête laser TL.

De plus, et comme le montre la figure 9, selon un autre mode de réalisation, la surface latérale tronconique 13 du corps 16 est dépolie afin de disperser un faisceau de traitement 14 qui aurait été pointé en dehors de la direction voulue et aussi de rendre visible le faisceau de visée.

Selon encore un autre mode de réalisation, comme le montre la figure 10, une région 30 de la surface latérale tronconique 13 du corps 16 de la lentille 12 peut être polie pour atteindre, par effet de réflexion totale, les parties postérieures de l'oeil. Dans ce but, la surface latérale 13 peut être rendue réfléchissante, par exemple par dépôt sur celle-ci d'un matériau approprié. Il est aussi possible de combiner ce mode de réalisation avec le précédent en dépolissant le reste de la surface latérale tronconique du corps 16.

De ce fait, lors d'un mauvais pointage du faisceau de traitement, c'est-à-dire en dehors de la région de guidage optique 30, on produit une dispersion du faisceau de traitement 14, ce qui évite les risques de brûlures au niveau des tissus non-visés de l'oeil et constitue une sécurité pour le patient.

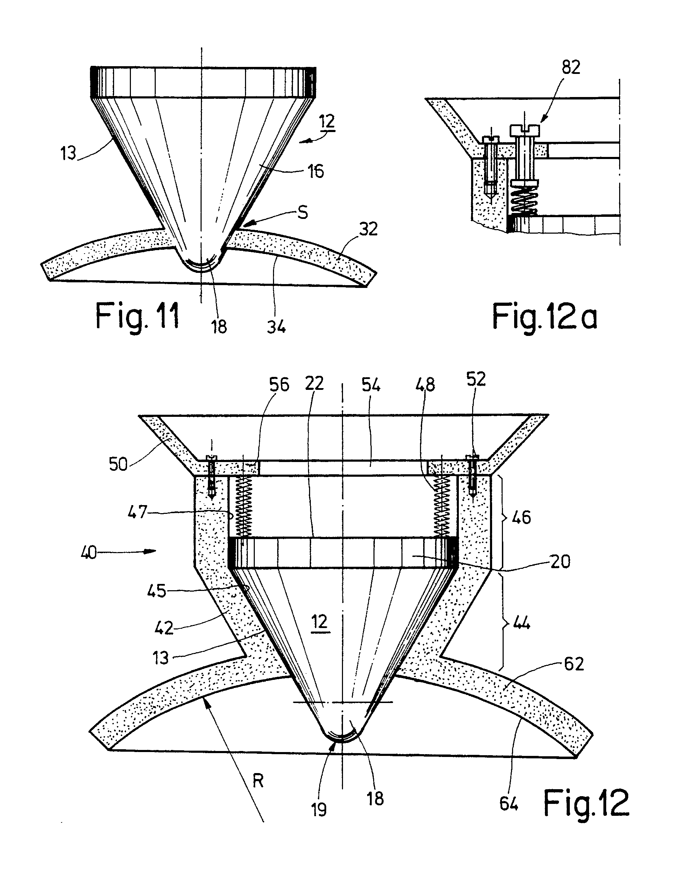

Selon encore un autre mode de réalisation représenté à la figure 11, on a disposé au niveau du sommet S du corps 16 de la lentille 12, c'est-à-dire au niveau de la jonction entre ce corps et l'élément protubérant 18, une embase 32 en forme de calotte ou coupole dont la surface intérieure sphérique concave 34 est conformée pour pouvoir épouser sensiblement la surface extérieure de l'oeil. De cette surface intérieure 34 fait saillie l'élément protubérant 18 qui présente une forme effilée pointée en direction de l'ouverture de l'embase 32. De préférence, cette embase 32 vient de matière avec le corps 16 et l'élément protubérant 18. Elle peut aussi être collée ou soudée sur la surface latérale 13 de la lentille. L'embase 32 peut être rendue au moins partiellement opaque aux radiations utilisées, l'élément protubérant 18 demeurant visible de l'extérieur de l'embase 32, il peut être précisément disposé sur l'oeil. Ce mode de réalisation présente l'avantage de pouvoir parfaitement positionner la lentille sur l'oeil et l'embase 32 forme, comme la surface annulaire 26 du mode de réalisation de la figure 5, un moyen de butée en profondeur et de positionnement de l'élément protubérant 18 sur la sclérotique 1.

Comme on le voit sur les figures 2, 12, 13 et 14, le verre de contact selon l'invention comporte de même un moyen support 40 dans lequel est emprisonnée et parfaitement maintenue la lentille optique 12.

Ce moyen support 40 permet la manipulation, notamment par un praticien, du verre de contact selon l'invention pour l'application de la lentille 12 sur la sclérotique 1. Selon les modes de réalisation représentés respectivement aux figures 2 et 12, le moyen support 40 est constitué d'une enceinte 42 formée d'une portion tronconique 44 surmontée d'une portion cylindrique 46. Ces deux portions respectivement cylindrique 46 et tronconique 44 sont conformées pour recevoir à l'intérieur de celles-ci une lentille 12 telle que celles représentées aux figures 3 à 10. La partie de guidage cylindrique 20 qui est ménagée sur la base de la lentille 12 vient parfaitement s'engager avec un léger jeu dans la portion cylindrique 46 de sorte que la lentille 12 peut se déplacer librement et axialement dans l'enceinte 42 du moyen support 40. A l'état de repos, comme cela est représenté à la figure 12, la lentille 12 vient reposer par sa surface latérale tronconique 13 contre la surface intérieure tronconique correspondante 45 de la région tronconique 44 qui forme siège, et ce sous l'action d'un ou de plusieurs organes élastiquement déformables 48. A cet effet, l'enceinte 42 du moyen support 40 comporte à sa base un flasque en forme de cuvette ou d'assiette 50 qui est de préférence relié de façon amovible à l'enceinte 42. Dans ce but, des éléments de fixation du type vis-écrou 52 assurent la liaison mécanique stable et amovible entre le flasque 50 et l'enceinte 42. Le flasque 50 comporte en son centre une ouverture 54 au travers de laquelle est destiné à passer le faisceau lumineux 14. La largeur de cette ouverture 54 est inférieure au diamètre de la surface interne 47 de la portion cylindrique 46 de l'enceinte 42, de sorte qu'un rebord 56 de ce flasque 50 recouvre partiellement la surface d'entrée 22 de la lentille 16.

Ainsi, le bord rentrant 56 forme un élément d'appui arrière pour les éléments élastiquement déformables 48 qui, dans ce mode de réalisation, sont constitués par des petits ressorts hélicoïdaux de compression répartis sur le pourtour de la surface 22.

On comprend donc que les deux modes de réalisation des figures 2 et 12 sont fonctionnellement identiques, et que dans ceux-ci la lentille optique 12 est apte à se translater longitudinalement et axialement à l'intérieur du moyen support 40, et plus particulièrement à l'intérieur de l'enceinte 42 à l'encontre de l'action élastique antagoniste des ressorts 48 lorsqu'une force est exercée sur l'extrémité libre 19 de l'élément protubérant 18. Dans le mode de réalisation de la figure 2, l'extrémité libre de la portion tronconique 44 forme une surface annulaire 60 destinée à venir s'appliquer directement sur la sclérotique 1 ou sur la conjonctive 7.

Dans le mode de réalisation de la figure 12, le sommet de la portion tronconique 44 comporte une embase 62 de forme et de caractéristiques similaires à l'embase 32 de la figure 11 et dont la surface 64, qui est destinée à venir se positionner sur la sclérotique 1, est de forme sphérique concave et de rayon de courbure R proche de celui de la région de l'oeil où se trouve la sclérotique 1. On remarquera que dans les deux modes de réalisation des figures respectivement 2 et 12, la lentille optique 12 à l'état de repos (figure 12) fait extérieurement saillie de l'enceinte 42 du moyen support 40. Plus particulièrement, l'élément protubérant 18 sort complètement des surfaces respectivement 60 et 64, ainsi qu'une partie du corps 16 de la lentille 12. Ainsi, on comprend qu'en utilisation, le praticien vient positionner l'élément protubérant 18 sur la sclérotique 1 ou sur la conjonctive 7, par exemple sensiblement au droit des tissus visés de l'oeil, puis vient appuyer manuellement par l'intermédiaire du moyen support 40 en direction de la sclérotique 1 qui, sous l'action de l'élément protubérant 18, se comprime et se déforme.

Ainsi, la sclérotique 1 passe de l'épaisseur E1 à l'épaisseur E2 (figure 2) et subit la pression exercée par l'embout protubérant 18 ce qui diminue considérablement la dispersion du faisceau lumineux qui la traverse et cela, comme on le suppose, grâce à un certain alignement des fibres que comprennent les tissus de la sclérotique.

Bien entendu, dans les deux modes de réalisation qui viennent d'être décrits à l'aide des figures 2 et 12, la lentille 12 aurait pu être immobilisée à l'intérieur du moyen support 40 en faisant sortir de l'enceinte 42 uniquement l'élément protubérant 18. Ainsi, en positionnant le moyen support 40, et plus particulièrement la face 60 ou la face 64 sur la sclérotique 1, l'élément protubérant 18 pourrait s'enfoncer dans la sclérotique jusqu'à une profondeur définie par la distance entre la surface 60 ou 64 et l'extrémité 19 de l'élément protubérant 18.

Toutefois, pour mieux contrôler la pression appliquée sur la sclérotique 1 et, en conséquence, pour éviter de l'endommager, il est préférable de rendre la lentille optique 12 mobile axialement à l'intérieur du moyen support 40 comme cela est représenté sur les figures 2 et 12, en interposant entre cette lentille 12 et le moyen support 40 des éléments élastiquement déformables 48. Ainsi, avec un choix judicieux des caractéristiques élastiques des ressorts 48, on peut choisir la pression maximale qui est appliquée sur l'oeil, et ce en fonction du type de sclérotique rencontrée. On a donc constitué par cet agencement un système de contrôle de la pression appliquée sur l'oeil, cette pression étant sensiblement constante· pour une course donnée de la lentille 12 dans le moyen support 40, quelle que soit la force avec laquelle le praticien appuie le moyen support 40 sur l'oeil.

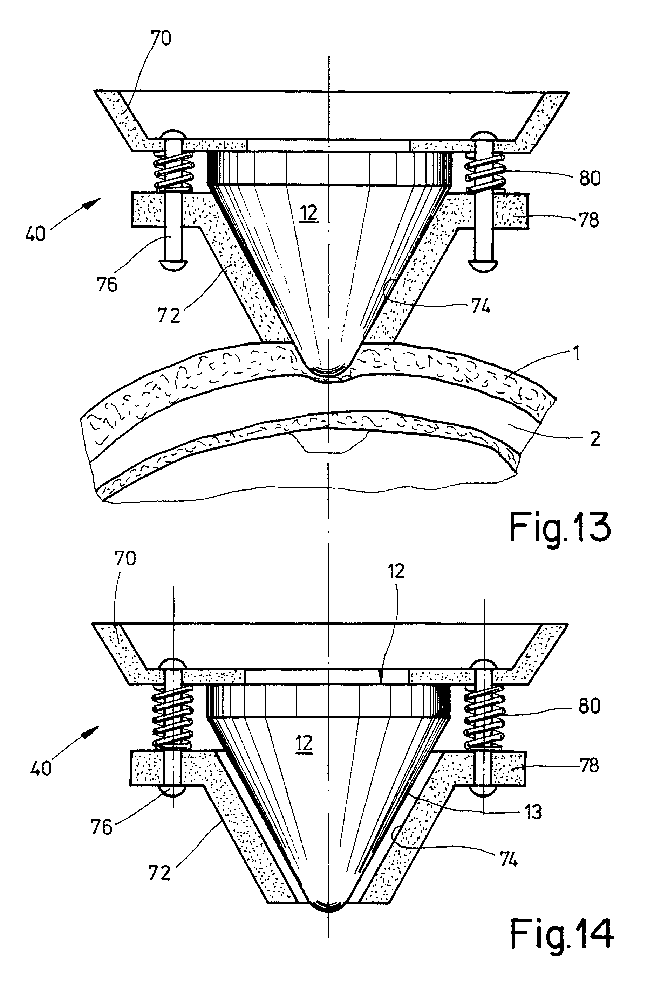

Dans un autre mode de réalisation représenté aux figures 13 et 14, l'élément support 40 est constitué de deux éléments 70 et 72 mobiles l'un par rapport à l'autre, l'élément 70 ayant sensiblement la même forme que le flasque 50. L'élément 72, quant à lui, comporte une surface intérieure tronconique 74 sur laquelle est destinée à venir reposer la surface latérale tronconique 13 correspondante de la lentille optique 12, mais cela en mode de fonctionnement comme cela est représenté sur la figure 13. Cette surface 74 forme une butée pour le déplacement axial de l'élément 72.

Pour pouvoir être parfaitement guidés axialement l'un par rapport à l'autre, les deux éléments 70 et 72 du moyen support 40 sont reliés entre eux par plusieurs axes 76 répartis sur le pourtour de ces éléments 70, 72. Les axes 76 présentent une longueur utile suffisante pour assurer le déplacement axial libre de l'élément 72 par rapport à l'élément 70. A cet effet, l'élément 70 présente une collerette 78 qui peut être continue ou réalisée sous la forme d'oreilles dans lesquelles viennent se loger les axes 76. Entre cette collerette 78 et le flasque 70 est disposé un ensemble d'éléments élastiquement déformables 80, ici constitués par des ressorts hélicoïdaux de compression. On remarque donc qu'à l'état de repos, tel que cela est représenté sur la figure 14, l'élément 72 est éloigné de la lentille optique 12 qui est fixement accolée sur l'élément 70, par exemple par collage. En utilisation, comme cela est représenté sur la figure 13, au fur et à mesure que l'élément protubérant 18 comprime la sclérotique 1, l'élément 72 qui appuie sur la sclérotique, recule à l'encontre de l'action des éléments élastiquement déformables 80 jusqu'à venir éventuellement rencontrer la surface latérale tronconique 13 de la lentille optique 12. Bien entendu, ce mode de réalisation pourrait comporter une embase 64, telle que représentée à la figure 12, pour permettre un meilleur positionnement et une meilleure assise de la lentille 12 sur l'oeil à traiter. On précisera que la longueur des axes 76 peut être prévue de sorte qu'à l'état de repos, l'extrémité 19 de la lentille soit cachée dans l'élément 72 afin de protéger l'élément protubérant 18.

Les systèmes à seuil de pression des figures 2 et 12 à 14 offrent une sécurité lors des traitements ophtalmologiques et permettent de ne pas atteindre le stade de déformation plastique de la sclérotique.

De préférence, le moyen support 40 est réalisé dans un matériau qui est non réfléchissant, qui est stérilisable et par exemple partiellement opaque ou qui peut être rendu au moins partiellement opaque. Ce matériau peut être un matériau métallique ou un matériau plastique, tel que ceux couramment utilisés en chirurgie. On précisera encore ici que les éléments élastiquement déformables peuvent être interchangés pour modifier la pression appliquée à la sclérotique par la lentille optique 12, ou être associés à des moyens d'ajustement de cette pression. Comme il est représenté sur la figure 12a de tels moyens peuvent être constitués par des systèmes vis-écrou 82 dont le filetage est engagé dans le bord rentrant 56 du flasque 50 et dont une extrémité appuie sur l'arrière de chaque élément élastique 48. De cette manière, on comprime plus ou moins l'élément 48 ce qui modifie, en fonction d'une course déterminée de la lentille optique 12 à l'intérieur du moyen support 40, la pression appliquée sur la sclérotique. Un tel moyen d'ajustement peut aussi être constitué par un empilement de rondelles interposées entre le bord rentrant 56 et les éléments élastiquement déformables 48. On comprend donc que dans les modes de réalisations des figures 2 et 12 à 14, le moyen support 40 ou au moins un élément 72 de celui-ci et la lentille optique 12 sont prévus mobiles l'un par rapport à l'autre avec interposition entre ceux-ci d'organes élastiquement déformables. On précisera ici que l'élément protubérant 18 ainsi que le corps 16 de la lentille optique 12 sont réalisés en un matériau qui doit être transparent pour des radiations de traitement peu ou pas absorbées par la sclérotique, par exemple d'une longueur d'onde de 700 à 1100 " $10^{-9}$ mètres (700-1100 nanomètres).

L'élément protubérant 18 qui vient en contact avec les tissus doit être stérilisable et ne doit pas réagir chimiquement avec ces tissus ainsi qu'avec le film liquide entre l'élément protubérant lui-même et les tissus. Il doit présenter un indice de réfraction proche de celui de l'eau pour minimiser la réflexion à l'interface entre l'embout protubérant 18 et les tissus, à savoir un indice de réfraction compris entre 1,3 et 1,7. A titre d'exemple, le matériau constituant l'élément protubérant 18, et de préférence le corps 16 lorsqu'ils viennent de matière, sont choisis parmi le verre du type BK7, un matériau cristallin tel que le saphir ou le grenat d'yttrium-aluminium (YAG), ou un matériau organique plastique du type polyméthacrylate de méthyle (PMMA).

La mise en oeuvre du verre de contact selon l'invention pour l'ophtalmothérapie transsclérotique / transchoroïdale est la suivante.

Selon un premier procédé, on dispose tout d'abord la lentille 12 sur l'oeil en mettant en appui directement l'élément protubérant 18 sur la conjonctive 7 ou sur la sclérotique 1. Ensuite, on pointe précisément le faisceau de visée sur la région visée de l'oeil au travers de la lentille 12.

Selon un deuxième procédé, on agit de façon inverse en disposant tout d'abord la tête laser TL au-dessus de l'oeil pathologique en émettant le faisceau lumineux de visée qui est visible et qui permet de pointer le faisceau laser sur la région de l'oeil à irradier. Ensuite, on dispose dans ce faisceau le verre de contact qui vient en appui sur la conjonctive ou sur la sclérotique en essayant de positionner l'axe a de façon normale à l'oeil du patient. Dans ces deux procédés, lorsque l'on estime que le verre de contact est parfaitement positionné, on remplace le faisceau de visée par un faisceau de traitement qui va atteindre le tissu à irradier et le traiter notamment par photocoagulation.

On remarquera que dans ce procédé de traitement, le faisceau lumineux 14 est émis librement de la tête laser TL, aucune liaison mécanique existant entre le verre de contact 40 et le dispositif laser utilisé. Le verre de contact selon l'invention étant maintenu manuellement par le praticien, par l'intermédiaire du moyen support 40, il est possible d'orienter, sur la sclérotique 1 qui est convenablement comprimée, l'axe longitudinal a de la lentille optique 12 afin de guider à l'intérieur de l'oeil le faisceau lumineux de traitement 14 de façon appropriée.

## Revendications

1. Verre de contact pour l'ophtalmothérapie transsclérotique / transchoroïdale à l'aide d'un faisceau lumineux, comprenant une lentille optique (12) comportant une surface d'entrée (22) du faisceau lumineux (14) et une surface de sortie (24) dont au moins une partie est destinée à venir en contact directement avec la conjonctive (7) ou la sclérotique (1) de l'oeil à traiter et par laquelle sort ledit faisceau lumineux (14), caractérisé en ce que la surface de sortie (24) de ladite lentille (12) comporte un élément protubérant (18, 18a, 18b, 18c) apte à transmettre ledit faisceau lumineux et destiné à venir comprimer ladite sclérotique (1) afin de diminuer son épaisseur à l'endroit du passage dudit faisceau lumineux (14).

2. Verre de contact selon la revendication 1, caractérisé en ce que ledit élément protubérant (18, 18a, 18b, 18c) a une surface frontale (Sf18) faible par rapport à la surface frontale de l'oeil à traiter.

3. Verre de contact selon la revendication 1 ou 2, caractérisé en ce que ledit élément protubérant (18, 18a, 18b, 18c) vient de matière avec ladite lentille optique (12).

4. Verre de contact selon la revendication 1, 2 ou 3, caractérisé en ce que ledit élément protubérant (18, 18a, 18b, 18c) et la lentille optique (12) forment une pièce monolithique de forme sensiblement tronconique.

5. Verre de contact selon l'une des revendications 1 à 4, caractérisé en ce que l'extrémité libre (19) de l'élément protubérant (18) est de forme sensiblement hémisphérique.

6. Verre de contact selon l'une des revendications 1 à 4, caractérisé en ce que l'extrémité libre (19) de l'élément protubérant (18a) est de forme sensiblement plane.

7. Verre de contact selon l'une des revendications 1 à 3, caractérisé en ce que l'élément protubérant (18b, 18c) a une forme au moins partiellement sphérique.

8. Verre de contact selon l'une des revendications 1 à 3, caractérisé en ce que l'élément protubérant (18b) est de surface frontale plus petite que celle de l'extrémité libre correspondante de la lentille (12) de sorte que la surface de cette extrémité libre de la lentille (12), en venant en appui sur la sclérotique (1) ou la conjonctive (7), forme butée et limite l'écrasement de la sclérotique.

9. Verre de contact selon l'une des revendications précédentes, caractérisé en ce qu'il comporte un moyen support (40) dans lequel est maintenu ladite lentille optique (12), ce moyen support (40) ou au moins un élément (72) de celui-ci et la lentille optique (12) étant mobiles l'un par rapport à l'autre.

10. Verre de contact selon la revendication 9, caractérisé en ce qu'il comporte un moyen (48, 80) de contrôle de la pression appliquée sur la sclérotique.

11. Verre de contact selon la revendication 10, caractérisé en ce que le moyen de contrôle de la pression appliquée sur l'oeil est constitué par au moins un organe élastiquement déformable (48, 80).

12. Verre de contact selon l'une quelconque des revendications prédédentes, caractérisé en ce que la lentille (12) comporte au moins un moyen (30) de guidage optique du faisceau lumineux.

13. Verre de contact selon l'une quelconque des revendications prédédentes, caractérisé en ce que la lentille (12) comporte sur sa surface latérale (13) un moyen de dispersion du faisceau lumineux constituant une sécurité pour le patient.

Fig.1

Fig.2

Fig.3

Fig.4

Fig.6

Fig.5

Fig.8

Fig.7

Fig.9

Fig.10

**Fig. 11**

**Fig. 12a**

**Fig. 12**

Fig.13

Fig.14

Office européen
des brevets

**RAPPORT DE RECHERCHE
EUROPEENNE**

Numéro de la demande

**EP 90 12 3719**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | GB-A-2 049 216   (KONAN CAMERA RESEARCH)<br>* Figures; page 1, lignes 72-100 *<br>— — — | 1,2,6 | A 61<br>F 9/00<br>A 61 B 3/125 |
| A | US-A-3 954 329   (POMERANTZEFF)<br>* Résumé; figures; colonne 5, ligne 55 - colonne 6, ligne 60 *<br>— — — | 1,2,6,10,<br>11 | |
| A | FR-A-2 255 616   (KRASNOV)<br>— — — | | |
| A | WO-A-8 912 424   (SONOMED)<br>— — — | | |
| A | DE-A-3 718 599   (RODENSTOCK)<br>— — — | | |
| D,A | EP-A-0 059 159   (LASAG AG)<br>— — — — — | | |

|  |
|---|
| **DOMAINES TECHNIQUES RECHERCHES (Int. Cl.5)** |
| A 61 F<br>A 61 B |

Le présent rapport de recherche a été établi pour toutes les revendications

| Lieu de la recherche | Date d'achèvement de la recherche | Examinateur |
|---|---|---|
| La Haye | 21 mars 91 | STEENBAKKER J. |